Europäisches Patentamt

European Patent Office

Office européen des brevets

(19)

(11) Veröffentlichungsnummer : **0 101 980**
**B1**

(12) **EUROPÄISCHE PATENTSCHRIFT**

(45) Veröffentlichungstag der Patentschrift :
22.02.89

(51) Int. Cl.⁴ : **G 01 N 33/52, C 12 Q  1/26**

(21) Anmeldenummer : **83107700.3**

(22) Anmeldetag : **03.08.83**

(54) **Verfahren zum Herstellen einer diagnostischen Vorrichtung.**

(30) Priorität : **23.08.82 DE 3231287**

(43) Veröffentlichungstag der Anmeldung :
**07.03.84 Patentblatt 84/10**

(45) Bekanntmachung des Hinweises auf die Patenterteilung : **22.02.89 Patentblatt 89/08**

(84) Benannte Vertragsstaaten :
**AT BE CH FR GB IT LI LU NL SE**

(56) Entgegenhaltungen :
EP–A– 0 051 709
GB–A–  893 301
US–A– 4 266 022

(73) Patentinhaber : **Eisai Co., Ltd.**
**6-10, Koishikawa 4-chome Bunkyo-ku**
**Tokyo 112 (JP)**

(72) Erfinder : **Roy, Asok Kumar, Dr.**
**Strünckweg 11**
**D-1000 Berlin 13 (DE)**
Erfinder : **Steinbach, Roland Wilfried**
**Lothringerstrasse 14**
**D-5000 Köln 1 (DE)**

(74) Vertreter : **Strehl, Schübel-Hopf, Groening, Schulz**
**Maximilianstrasse 54 Postfach 22 14 55**
**D-8000 München 22 (DE)**

EP 0 101 980 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zum Herstellen einer diagnostischen Vorrichtung, die insbesondere zum Nachweis einer pathologisch erhöhten Konzentration von Dehydrogenasen in Flüssigkeiten von Menschen, Tieren oder Pflanzen vorgesehen ist und aus einem Träger und einem Stoffgemisch besteht.

Medizinische Diagnosen spielen im Gesundheitswesen eine erhebliche Rolle. Dies gilt insbesondere für Diagnosen im Rahmen von Vorsorgeuntersuchungen. Dabei haben Diagnosen, z. B. zur Früherkennung von Malignomen, bereits eine große praktische Bedeutung erlangt, da bei einer rechtzeitigen Erkennung einer solchen Erkrankung die therapeutischen Erfolgsaussichten am größten sind.

Pathologische Veränderungen bei Menschen, Tieren oder Pflanzen können die Zusammensetzung extrazellulärer Flüssigkeiten dieser Lebewesen beeinflussen. Beispielsweise gehen bei pathologischen Veränderungen von Zellen, wie es bei der Bildung von Malignomen der Fall ist, Dehydrogenasen ins Serum und in andere extrazelluläre Flüssigkeiten. Dabei kann für jede Dehydrogenase jeweils ein als pathologisch zu betrachtender, diagnostisch verwertbarer Schwellenwert gefunden werden.

Es ist bereits möglich, chemische Diagnosemittel anzugeben, die einen an den pathologischen Schwellenwert einer Dehydrogenase angepaßten Umschlagpunkt aufweisen. Das Überschreiten dieses Umschlagspunktes kann z. B. mittels eines in dem diagnostischen Mittel enthaltenen Redox-Farbstoffes sichtbar gemacht werden, der beim pathologischen Schwellenwert von farblos nach farbig umschlägt.

Ein Diagnosemittel, das zum Nachweis einer pathologisch erhöhten Konzentration von Dehydrogenasen in extrazellulären Flüssigkeiten vorgesehen ist, enthält z. B. ein auf einen pH-Wert im sauren Bereich eingestelltes Stoffgemisch aus dem der jeweiligen Dehydrogenase entsprechenden Substrat, einer wasserstoffübertragenden Verbindung und mindestens einem Redox-Farbstoff. Damit das Mittel für eine Differentialdiagnostik geeignet ist, bei der nur pathologisch erhöhte Dehydrogenasekonzentrationen erfaßt werden, muß der genannte pH-Wert ziemlich niedrig, z. B. unter 5,0, liegen.

Für bestimmte Diagnosearten, wie intrakorporale Untersuchungen, ist es erforderlich, das diagnostische Mittel vor seiner Verwendung auf einen Träger aufzubringen. Dies gilt z. B. für Diagnosen bei Frauen, die auf das Vorliegen von Karzinomen im Genitalbereich überprüft werden sollen. Zu diesem Zweck ist aus der DE-PS 24 43 741 (die der US-A-4 266 022 entspricht) bekannt, das diagnostische Mittel auf einen Hygiene-Tampon aufzubringen und in dieser Form in die Vagina der Patientin, einzuführen, wo das Mittel mit im Vaginalsekret enthaltenen Dehydrogenasen reagieren kann. Zeigt der Tampon nach seiner Entnahme aus der Vagina eine Verfärbung, ist dies ein Hinweis auf eine pathologische Veränderung im Körper der Patientin.

Aus der DE-PS 24 43 741 ist unter anderem ein Verfahren zum Herstellen einer diagnostischen Vorrichtung bekannt, die aus einem Tampon und einem darauf aufgebrachten Stoffgemisch besteht. Die Vorrichtung ist zur gemeinsamen Bestimmung der Isoenzyme 4 und 5 der Lactatdehydrogenase vorgesehen und soll letzlich zur Diagnose von pathologischen Veränderungen bei Frauen im unteren Genitaltrakt dienen.

Bei dieser bekannten Herstellung wird jeweils unter Ausschluß von energiereichem Licht das auf einen pH-Wert im sauren Bereich eingestellte Stoffgemisch in flüssiger Form mit dem Tampon in Kontakt gebracht. Anschließend wird die Kombination aus Tampon und Stoffgemisch getrocknet und dann in einer für energiereiches Licht undurchlässigen Verpackungshülle eingeschlossen. Dabei wird besonders darauf geachtet, daß kein energiereiches Licht an die Vorrichtung gelangt. « Energiereiches Licht » bedeutet in diesem Zusammenhang und auch nachfolgend solches Licht (einschließlich UV-Licht), das energiereicher als Rotlicht ist.

In der Praxis hat sich herausgestellt, daß bestimmte Eigenschaften der diagnostischen Vorrichtung besonders wünschenswert sind.

Der Erfindung liegt daher die Aufgabe zugrunde, ein Verfahren zum Herstellen einer diagnostischen Vorrichtung anzugeben, die vor ihrer Verwendung möglichst lange Zeit gelagert werden kann und dabei ihre Funktionsfähigkeit nicht wesentlich verändert ; z. B. soll die Vorrichtung, falls sie einen Redox-Farbstoff enthält, während des Lagerns nicht zum vorzeitigen Verfärben neigen. Weiterhin soll die diagnostische Vorrichtung während einer möglichst kurzen Anwendungszeit (Liegezeit) beim Patienten eine hohe diagnostische Treffsicherheit erreichen. Auch wird für den Fall, daß das diagnostische Mittel bei seiner intrakorporalen Anwendung einen Farbstoff bildet, eine möglichst feste Bindung des Farbstoffs an den Träger der diagnostischen Vorrichtung angestrebt, um ein Übertreten dieses Farbstoffs z. B. in die Vaginalhaut einer untersuchten Frau zu vermeiden. Schließlich soll der Zeitaufwand für die Herstellung der diagnostischen Vorrichtung verkürzt werden.

Diese Aufgabenstellung wird erfindungsgemäß gelöst durch ein Verfahren zum Herstellen einer diagnostischen Vorrichtung zum Nachweis einer pathologisch erhöhten Konzentration von Dehydrogenasen bei Menschen, Tieren oder Pflanzen, die aus einem Träger und einem Stoffgemisch besteht, wobei jeweils unter Ausschluß von energiereichem Licht das auf einen pH-Wert im sauren Bereich eingestellte Stoffgemisch in flüssiger Form mit dem Träger in Kontakt gebracht, getrocknet und dann in einer für energiereiches Licht undurchlässigen Verpackungshülle eingeschlossen wird. Das Verfahren ist dadurch gekennzeichnet, daß die Kombination aus Träger und Stoffgemisch bis auf einen Feuchtigkeitsgehalt

2

EP 0 101 980 B1

unter 1 % getrocknet und in der Verpackungshülle gegen Zutritt von Luft und Feuchtigkeit gesichert verschlossen wird.

Die erfindungsgemäß hergestellte Vorrichtung kann vor ihrer Verwendung mehr als 3 1/2 Jahre ohne Einbuße ihrer Lagerfähigkeit gelagert werden. Außerdem ist die Anwendungszeit deutlich verkürzt ; sie beträgt maximal 10 min., im Vergleich zu 15 bis 30 min. mit der Vorrichtung gemäß DE-PS 24 43 741, wodurch die Gefahr, daß Substanzen der Vorrichtung in den Körper gelangen, minimal ist.

Gemäß einer bevorzugten Ausführungsform des Verfahrens werden ein Träger, der einen Redox-Farbstoff bindet, sowie ein Stoffgemisch aus dem der jeweiligen nachzuweisenden Dehydrogenase entsprechenden Substrat, einer wasserstoffübertragenden Verbindung, mindestens einem Redox-Farbstoff und gegebenenfalls einem Puffersystem eingesetzt, wobei das Verfahren dadurch gekennzeichnet ist, daß der Träger polare Gruppen aufweist sowie das Stoffgemisch auf einen pH-Wert von unter 5,0 eingestellt ist. Damit wird eine diagnostische Vorrichtung erhalten, die sich insbesondere für eine Differentialdiagnostik zum Nachweis von pathologisch erhöhten Dehydrogenasekonzentrationen eignet und neben einer langen Lagerfähigkeit längere Zeit, z. B. mehr als zwei Jahre, eine hohe diagnostische Treffsicherheit aufweist.

Bei einer besonderen Weiterbildung des Verfahrens wird als Träger ein Cellulosefasern enthaltender Tampon eingesetzt. Die so hergestellte diagnostische Vorrichtung bewährt sich vor allem für intrakorporale Untersuchungen, wie Diagnosen von Karzinomen im Genitalbereich von Frauen. Diese diagnostische Vorrichtung ist vor ihrer Verwendung mehrere Jahre lagerfähig, erreicht trotz einer kurzen Liegezeit in der Patientin von maximal 10 Minuten eine hohe diagnostische Treffsicherheit und garantiert eine feste Bindung des bei der Diagnose gegebenenfalls gebildeten Farbstoffs an die Cellulosefasern des Tampons, so daß ein Übertreten des Farbstoffs in die Vaginalschleimhaut der untersuchten Patientin unterbleibt.

Tampons aus Cellulosefasern haben sich für solche diagnostischen Vorrichtungen besonders bewährt, die zur Feststellung von Karzinomen im weiblichen Genitalbereich vorgesehen sind. Es können dazu übliche Hygienetampons verwendet werden. Diese sind vorzugsweise nicht gewickelt, sondern weisen eine Längsnaht auf. Die Tampons aus Cellulosefasern haben den besonderen Vorteil, daß sie den bei der Verwendung der diagnostischen Vorrichtung gebildeten Farbstoff besonders stark binden, wobei die polaren Gruppen des Cellulosematerials und entsprechende polare Gruppen im Farbstoff vermutlich eine feste Bindung eingehen.

Die Stärke der Bindung zwischen den Cellulosefasern und dem Farbstoff zeigen Experimente, bei denen versucht worden ist, den Farbstoff (Formazan) aus dem Tampon herauszulösen. Als Extraktionsmittel dienten vier verschiedene Medien :

a. physiologische Kochsalzlösung,
b. eine Lösung, die zur Imitation von Vaginalsekret (das nicht in ausreichender Menge erhältlich ist) Humanalbumin und Mucin enthielt,
c. eine Lösung gemäß b., die zusätzlich einen grenzflächenaktiven Stoff, z. B. Natriumlaurylsulfat, umfaßte, sowie
d. Chloroform.

Mit keinem dieser Medien konnten größere Mengen des Farbstoffs (Formazan) aus dem Tampon herausgelöst werden. Selbst durch sechstündiges Erhitzen des Tampons in siedendem Chloroform waren nur etwa 7 % des Farbstoffs (Formazan) extrahierbar. Da der Tampon bei der Verwendung in der Humanmedizin nur maximal 10 Minuten bei etwa 37 °C in einer Körperhöhle verbleibt, besteht offensichtlich keine Gefahr, daß ein Teil des gegebenenfalls gebildeten Farbstoffs von der den Tampon benetzenden Körperflüssigkeit extrahiert wird und in den Körper gelangt.

Der Tampon kann auch aus anderen Fasern, z. B. hydrophilen Kunstfasern, bestehen, die zur Vergrößerung ihrer Oberfläche und ihres Absorptionsvermögens hohl ausgebildet sein können. Selbstverständlich sind Tampons aus einem Gemisch verschiedener Faserarten, z. B. natürlichen und synthetischen Fasern, einsetzbar, solange die Fasern hydrophil sind und polare Gruppen aufweisen.

Die einen Tampon umfassende diagnostische Vorrichtung eignet sich nicht nur zur intrakorporalen, sondern auch zur extrakorporalen Diagnose. Im letzteren Fall wird die zu untersuchende Körperflüssigkeit mit dem Bereich des Tampons in Berührung gebracht, der das erwähnte Stoffgemisch enthält.

Vorzugsweise wird die Kombination aus dem Träger und dem Stoffgemisch bereits vor dem Trocknen in der Verpackungshülle angeordnet. Auf diese Weise braucht nach dem Trocknen die Verpackungshülle nur noch verschlossen zu werden. Da dieses Verschließen nur kurze Zeit dauert, vermeidet man so die Gefahr, daß erneut Feuchtigkeit an den Träger und das Stoffgemisch gelangt und die Haltbarkeit der diagnostischen Vorrichtung beeinträchtigt.

Gemäß einer besonderen Ausführungsform wird die Verpackungshülle, die den Träger und das Stoffgemisch enthält, noch in der Trocknungsvorrichtung verschlossen.

Dadurch wird gewährleistet, daß der durch das Trocknen erreichte niedrige Feuchtigkeitsgehalt auch nach dem Verschließen der Verpackungshülle in deren Innenraum vorliegt.

Für die Verwendung der diagnostischen Vorrichtung in Form eines Tampons ist es günstig, wenn der Tampon in einem Applikator angeordnet ist. Damit wird erreicht, daß der Tampon ohne unmittelbare Berührung mit den Fingern und ohne Gefahr einer Infizierung in eine Körperhöhle, z. B. in die Vagina, eingeführt werden kann.

Dementsprechend wird bei der Herstellung einer solchen diagnostischen Vorrichtung der Tampon in die Einführhülse des Applikators eingebracht. Dabei ist es bevorzugt, den Tampon vor dem Aufbringen des Stoffgemisches in dem Applikator anzuordnen, weil dann der Applikator als Halterung für den Tampon dient, wenn auf diesen das Stoffgemisch aufgebracht wird.

Es ist vorteilhaft, wenn der Applikator eine Einführhülse mit einer flüssigkeitsundurchlässigen Innenwand aufweist, und der Tampon derart in der Einführhülse angeordnet wird, daß innerhalb der Einführhülse vor dem Einführende des Tampons ein freier Raum gebildet wird. Dieser Raum kann vorübergehend das auf den Tampon aufzubringende Stoffgemisch aufnehmen und trägt so zu einem beschleunigten Verfahrensablauf bei. Die flüssigkeitsundurchlässige Innenwand der Einführhülse des Applikators ermöglicht ein rasches Aufgeben des Stoffgemisches in flüssiger Form auf den Tampon, ohne daß ein Teil dieser Flüssigkeit von der Wand der Einführhülse aufgesaugt wird oder gar durch sie hindurchtritt und so für den Tampon verloren geht.

Bei einer für die Praxis besonders geeigneten Ausführungsform des Verfahrens wird eine Lösung des Stoffgemisches in den erwähnten freien Raum eingespritzt, z. B. mittels einer Dosiervorrichtung. Dieses Einspritzen kann sehr rasch erfolgen, wodurch die Produktionsgeschwindigkeit erhöht werden kann.

Als Verpackungshülle wird vorzugsweise ein einseitig offenes, aus Metall hoher Wärmeleitfähigkeit bestehendes Röhrchen mit einem elastischen Verschlußstopfen eingesetzt. Ein solches Röhrchen, z. B. aus Aluminium, ist seinerseits leicht herstellbar und gewährleistet nach dem Aufsetzen des Verschlußstopfens, der aus einem gummiartigen Material bestehen kann, einen dauerhaften Schutz der diagnostischen Vorrichtung vor Luft, Feuchtigkeit, Licht und mechanischer Beschädigung.

Außerdem kann das Röhrchen als Halterung für die diagnostische Vorrichtung während des Aufbringens des Stoffgemisches auf den Träger dienen.

Schließlich begünstigt die hohe Wärmeleitfähigkeit des Röhrchenmaterials einen raschen Wärmetransport zwischen den einzelnen Röhrchen. Dies ist vorteilhaft, wenn die Röhrchen, nachdem sie jeweils mit einer diagnostischen Vorrichtung beschickt worden sind, unter gegenseitiger Berührung mit ihrem Inhalt getrocknet werden.

Bei einer Weiterbildung dieser Verfahrensvariante wird das Röhrchen mit der Kombination aus Applikator und Tampon beschickt, dann das Stoffgemisch auf den Tampon aufgebracht sowie anschließend das Röhrchen mit dem Verschlußstopfen teilweise verschlossen und mit seinem Inhalt getrocknet. Das teilweise Verschließen des Röhrchens ermöglicht einerseits das gewünschte Trocknen und andererseits nach dem Trocknen ein rasches vollständiges Verschließen des Röhrchens, was sogar noch innerhalb der Trocknungsvorrichtung geschehen kann.

Zur Erzielung einer besonders langen Haltbarkeit der verpackten diagnostischen Vorrichtungen werden der Träger und das Stoffgemisch in einer Gefriertrocknungsvorrichtung getrocknet, und anschließend wird dort die Verpackungshülle unter vermindertem Druck, der für den Trocknungsvorgang in der Gefriertrocknungsvorrichtung bereits eingestellt war, verschlossen.

Das Gefriertrocknen ist für das vorliegende Verfahren besonders empfehlenswert, da das Trockengut verhältnismässig rasch und weitgehend von dem Lösungsmittel für das Stoffgemisch befreit wird.

Ein rasches Trocknen begünstigt eine sehr feine Verteilung des Stoffgemisches auf dem Träger. Das bedeutet, daß das Stoffgemisch nach dem Trocknen hauptsächlich in amorpher Form und nicht in Form größerer Kristalle an den Träger gebunden ist. Die Bindung zwischen den einzelnen Bestandteilen des Stoffgemisches, insbesondere der Farbstoffkomponente, und dem Träger wird dadurch erhöht.

Außerdem werden beim Gefriertrocknen auch dem Träger, z. B. den Cellulosefasern des Tampons, flüchtige Bestandteile, z. B. Wasser, entzogen, wodurch für die Moleküle der Farbstoffkomponente die räumlichen Bedingungen noch verbessert werden, um einen möglichstengen Kontakt mit den Molekülen des Trägers einzugehen. Die Folge sind eine größere Anzahl kovalenter Bindungen zwischen Farbstoff und Träger. Dadurch wird die Gefahr des Herauslösens von Farbstoffmolekülen aus dem Träger durch Körperflüssigkeiten stark herabgesetzt.

Außerdem hat sich gezeigt, daß durch die feste Bindung zwischen dem Träger und dem Farbstoff die bei der Verwendung der diagnostischen Vorrichtung ablaufende Farbreaktion bei einem erwünschten pH-Wert von unter 5,0 besonders spezifisch verläuft und dadurch die Treffsicherheit der Diagnose beträchtlich erhöht wird.

Die durch das Gefriertrocknen erreichte starke Bindung zwischen Stoffgemisch und Träger ist auch ursächlich für die lange Haltbarkeit der derart hergestellten diagnostischen Vorrichtungen.

Die diagnostische Vorrichtung wird optimal gefriergetrocknet, wenn in einer ersten Stufe bei einem geringeren Unterdruck und in einer zweiten Stufe bei einem größeren Unterdruck getrocknet wird. Dies begünstigt ein Abtrennen von z. B. Wassermolekülen, die unabhängig vom aufgebrachten Stoffgemisch im Träger enthalten sind. Der geringere Unterdruck für das Vortrocknen liegt in der Größenordnung von etwa 1,33 Pa ($10^{-2}$ Torr). In der anschließenden Stufe des Nachtrocknens wird der größere Unterdruck auf etwa 0,13 Pa ($10^{-3}$ Torr) eingestellt.

Nachfolgend werden das erfindungsgemässe Verfahren und die Dauer der Haltbarkeit von derart hergestellten diagnostischen Vorrichtungen beispielhaft erläutert.

4

## Beispiel 1

Die einzelnen Komponenten des Stoffgemisches werden eingewogen und unter Ausschluß von energiereichem Licht, z. B. unter Rotlicht, in destilliertem Wasser gelöst. Der Ausschluß von energiereichem Licht wird auf für die nachfolgenden Verfahrensstufen sichergestellt.

Der Träger für das Stoffgemisch ist ein üblicher Hygienetampon aus Cellulosefasern. Er wird in der Einführhülse eines Tamponapplikators angeordnet, in die eine Ausstoßhülse mit etwas kleinerem Außendurchmesser teleskopartig teilweise eingeschoben ist. Beide Hülsen des Applikators sind z. B. aus gewickeltem Karton oder Kunststoff hergestellt. Solche Applikatoren sind handelsüblich.

Mittels einer Einstanzung in dem Bereich, wo sich die Einführhülse und die Ausstoßhülse des Applikators überlappen, wird ein unbeabsichtigtes Herausgleiten der Ausstoßhülse aus der Einführhülse verhindert.

Der Tampon wird über das Einführende der Einführhülse so weit in den Applikator eingeschoben, daß er am Vorderende der Ausstoßhülse anliegt und gleichzeitig innerhalb der Einführhülse vor dem Einführende des Tampons ein freier Raum von etwa 3 cm³ bzw. mit einer axialen Länge von etwa 3 cm gebildet wird. Der Rückholfaden des Tampons erstreckt sich vom rückwärtigen Tamponende innerhalb des Ausstoßhülse bis über deren rückwärtiges Ende hinaus.

Die Anordnung aus Applikator und Tampon wird derart in ein Verpackungsröhrchen aus Aluminium eingebracht, daß das Einführende des Applikators und die Öffnung des Röhrchens nach oben gerichtet sind.

Anschließend werden mittels einer Dosiervorrichtung von oben in den genannten freien Raum innerhalb der Einführhülse und vor dem Einführende des Tampons etwa 2 ml der eingangs hergestellten wäßrigen Lösung des Stoffgemisches eingespritzt.

Zur Erhöhung der Produktionsgeschwindigkeit erfolgt dieses Einspritzen so rasch, daß für kurze Zeit ein Teil der Lösung in dem erwähnten freien Raum des Applikators stehen bleibt, bevor sie von dem Tampon ganz aufgesaugt ist. Um zu verhindern, daß während dieser Zeit und auch noch nach dem Aufsaugen der Lösung ein Teil hiervon in die wasserlösliche oder wasseraufnehmende Wandung der Einführhülse eindringt und so für den Tampon verloren geht, ist die Einführhülse an ihrer Innenwand mit einem flüssigkeitsundurchlässigen Stoff, z. B. einer Aluminium- oder PVDC-Folie, beschichtet.

Die Lösung des Stoffgemisches verursacht ein Quellen des Tampons innerhalb des Applikators. Da der Tampon bereits am Einführende der Ausstoßhülse anliegt und durch dessen Festlegung mittels der erwähnten Einstanzung eine Ausdehnung des Tampons zum rückwärtigen Applikatorende verhindert wird, weicht der Tampon bei seiner Volumenvergrößerung durch Flüssigkeitsaufnahme in Richtung des Einführendes des Applikators aus und füllt dabei im wesentlichen den genannten freien Raum in dessen Einführhülse aus.

Anschließend wird auf die Öffnung des Verpackungsröhrchens ein Stopfen aus einem elastischen Material teilweise aufgesetzt. Dabei wird der Stopfen bis zu einem ersten Vorsprung an seinem Umfang in das Röhrchen eingedrückt. Bei dieser Position des Stopfens bleibt mittels einer Ausnehmung im Stopfen ein Strömungskanal zwischen dem Innenraum des Röhrchens und seiner äußeren Umgebung frei.

Eine Vielzahl derart vorbereiteter Verpackungsröhrchen wird unter gegenseitiger Berührung nebeneinander gestellt und in eine Gefriertrocknungsvorrichtung gebracht.

Dort werden die Röhrchen mit ihrem Inhalt vorgefroren und anschließend während einer ersten Trocknungsstufe bei einem Unterdruck von etwa 1,33 Pa vorgetrocknet. In einer zweiten Trocknungsstufe wird das Trockengut bei einem größeren Unterdruck von 0,13 Pa nachgetrocknet. Das gesamte Trocknen dauert etwa 24 Stunden.

Wenn eine Restfeuchtigkeit von unter 1 % erreicht ist, werden die Verpackungsröhrchen in der Gefriertrocknungsvorrichtung unter dem bestehenden Unterdruck verschlossen. Dies geschieht durch ein Hydraulisches Absenken einer Verschlußplatte, welche die Verschlußstopfen der Verpackungsröhrchen bis zu einem zweiten Vorsprung am jeweiligen Stopfenumfang eindrückt und dadurch die Röhrchen vollständig verschließt.

Anschließend können die Verpackungsröhrchen aus der Gefriertrocknungsanlage entnommen und den Umgebungsbedingungen, wie Luft, üblicher Feuchtigkeit und energiereichem Licht, ausgesetzt werden, ohne die verpackten diagnostischen Vorrichtungen zu beeinträchtigen.

Nachfolgend werden spezielle Beispiele hinsichtlich der Zusammensetzung des Stoffgemisches und der Haltbarkeit der hergestellten diagnostischen Vorrichtungen angegeben.

## Beispiel 2

Gemäß Beispiel 1 werden diagnostische Vorrichtungen hergestellt. Als Träger dient ein üblicher Hygienetampon, der aus Cellulosefasern besteht und nicht gewickelt ist, sondern aus mehreren Cellulosefaserschichten besteht, welche zusammen mit einem Rückholband in Längsrichtung des Tampons miteinander vernäht und anschließend zur endgültigen Tamponform gepreßt worden sind.

Das Stoffgemisch wird in Form einer wäßrigen Lösung folgender Zusammensetzung auf den Tampon aufgebracht :

EP 0 101 980 B1

| Bestandteil | Menge |
|---|---|
| DL-Natriumlactat | 57,6 mg |
| Nicotinamid-adenindinucleotid | 3,0 mg |
| 2,2′-Azino-bis [3,3′-äthyl-benzthiazolin-6,6′-sulfonsäure] (MIT) | 272,5 µg |
| Triäthanolamin | 39,8 mg |
| Phenazinmethosulfat (PMS) | 10,0 µg |
| Wasser, gereinigtes ad | 2,0 g |

Der pH-Wert der Lösung wird mit 3n Salzsäure auf 4,0 eingestellt.

Nach dem genannten Verfahren werden diagnostische Vorrichtungen erhalten, die noch nach mehreren Jahren funktionsfähig sind.

Beispiel 3

Gemäß Beispiel 1 werden diagnostische Vorrichtungen unter Verwendung einer wäßrigen Lösung des Stoffgemisches mit folgender Zusammensetzung hergestellt :

| Bestandteil | Menge |
|---|---|
| DL-Natriumlactat | 57,6 mg |
| NAD | 3,0 mg |
| Nitroblau-tetrazoliumchlorid (NBT) | 556,0 µg |
| Diaphorase | 6,4 IE* |
| Triäthanolamin | 39,8 mg |
| Wasser, gereinigtes ad | 2,0 g |

* Internationale Einheiten

Der pH-Wert zur Lösung wird auf 4,9 eingestellt.

Die mit dieser Lösung erhaltenen diagnostischen Vorrichtungen sind in gleicher Weise verwendbar und haltbar wie die gemäß Beispiel 2 hergestellten Vorrichtungen.

Beispiel 4

Gemäß Beispiel 1 werden diagnostische Vorrichtungen unter Verwendung einer wäßrigen Lösung des Stoffgemisches folgender Zusammensetzung hergestellt :

| Bestandteil | Menge |
|---|---|
| DL-Natriumlactat | 57,6 mg |
| NAD | 3,0 mg |
| NBT | 50,0 µg |
| Meldolablau | 2,5 µg |
| Triäthanolaminhydrochlorid | 50,0 mg |
| Wasser, gereinigtes ad | 2,0 g |

Der pH-Wert der Lösung beträgt 4,0.

Die mit der Lösung erhaltenen diagnostischen Vorrichtungen haben die gleichen Eigenschaften wie die gemäß den Beispielen 2 und 3 hergestellten Vorrichtungen.

Beispiel 5

Gemäß Beispiel 1 werden diagnostische Vorrichtungen unter Verwendung einer wäßrigen Lösung des Stoffgemisches mit folgender Zusammensetzung hergestellt :

| Bestandteil | Menge |
|---|---|
| DL-Natriumlactat | 37,0 mg |
| NAD | 2,0 mg |
| NBT | 490,0 µg |
| PMS | 48,7 µg |
| Triäthanolamin-HCl | 49,0 mg |
| Wasser, gereinigtes ad | 2,019 g |

Die Lösung wird mit 3n Salzsäure auf einen pH-Wert von 4,5 eingestellt.

Die erhaltenen diagnostischen Vorrichtungen entsprechen in ihren Eigenschaften den Vorrichtungen gemäß den Beispielen 2 bis 4.

6

Beispiel 6

| Bestandteil | Menge |
|---|---|
| DL-Natriumlactat | 25,6 mg |
| NAD | 1,33 mg |
| Triphenyltetrazoliumchlorid (TNBT) | 555,0 µg |
| PMS | 30,3 µg |
| Triäthanolamin-HCl | 49,0 mg |
| Wasser, gereinigtes ad | 2,0 g |

Die Lösung wird mit 3n-HCl auf einen pH-Wert von 4,0 eingestellt.

Beispiel 7

Gemäß Beispiel 1 hergestellte diagnostische Vorrichtungen werden verschieden lange im verpackten Zustand gelagert und dann auf ihre Funktionsfähigkeit überprüft.

Die Tampons werden nach der entsprechenden Lagerzeit im Dunkeln aus ihrer Verpackung entnommen und jeweils in einem Reagenzglas mit 2 ml einer Testlösung in Berührung gebracht.

In allen Fällen handelt es sich bei der Testlösung um eine Phosphatpufferlösung mit einem pH-Wert von 7,5. Ein erster Teil dieser 2-ml-Proben der Testlösung wird als reine Phosphatpufferlösung zur Ermittlung der Blindwerte der verschieden alten Tampons herangezogen. Ein zweiter Teil der 2-ml-Proben der Testlösung wird zusätzlich mit jeweils drei Internationalen Einheiten (IE) Lactatdehydrogenase (LDH) versetzt. Ein dritter Teil der 2-ml-Proben der Testlösung wird mit jeweils 1 % Albumin, 0,9 % Natriumchlorid und 0,1 % Mucin modifiziert. Einem vierten Teil der 2-ml-Proben der Testlösung werden außer dem Albumin, dem Natriumchlorid und dem Mucin in den vorstehend genannten Mengen noch drei IE von LDH zugefügt.

Bei dieser Überprüfung der Funktionsfähigkeit der Tampons in vitro stellen sämtliche vorgenannten 2-ml-Proben der Testlösung jeweils in bezug auf ihre Zusammensetzung eine Imitation einer Körperflüssigkeit dar.

Die gesamte Überprüfung wird bei Raumtemperatur und unter Ausschluß von energiereichem Licht durchgeführt. Jede Probe (Tampon und Testlösung im Reagenzglas) wird 20 Minuten nach dem Benetzen des Tampons mit der Testlösung hinsichtlich der Intensität einer möglichen Verfärbung des Tampons visuell beurteilt und auch fotografiert, um diese Beurteilung später überprüfen zu können.

Die Ergebnisse sind in der nachfolgenden Tabelle zusammengefaßt :

Tabelle

| Tampon | | Testlösung | | | |
|---|---|---|---|---|---|
| pH-Wert des Stoffgemisches | Lagerzeit | nur Puffer (Blindprobe) | Puffer, LDH | Puffer, Albumin, NaCl, Mucin | Puffer Albumin, NaCl, Mucin, LDH |
| 4,5 | 1 Jahr | (–) | + | | |
| 4,5 | 1 Jahr | (–) | +bis++ | (–)bis gelb | +bis++ |
| 4,5 | 1 1/2 Jahre | (–) | +bis++ | (–) | +bis++ |
| 4,5 | 2 Jahre | (–) | + | | |
| 4,5 | 2 1/2 Jahre | (–) | (+)bis+ | | |
| 4,5 | 3 Jahre | (–) | +bis++ | | |
| 4,5 | 3 1/2 Jahre | (–) | +bis++ | (–) | (+)bis+ |

(—) = keine Verfärbung = Diagnose negativ
(+) = rosa = Diagnose noch negativ
+ = hellblau mit violettem Stich = Diagnose positiv
++ = blau = Diagnose positiv

7

Daraus ist ersichtlich, daß die diagnostische Vorrichtung mit einem Tampon als Träger auch nach dreieinhalb Jahren Lagerzeit noch funktionsfähig ist. Dies gilt auch unabhängig davon, ob die Tampons nur aus Cellulosefasern oder einem Gemisch aus Baumwolle und Cellulosefasern oder nur aus Baumwollfasern bestehen.

Der erfindungsgemäß eingesetzte Träger muß nicht unbedingt aus Fasern bestehen. Beispielsweise kann er auch aus einem Schaumstoff oder einer aufgewickelten oder in anderer Weise geformten Folie zusammengesetzt sein. Es ist lediglich erforderlich, daß der Träger ausreichend hydrophil ist, damit er bei der Verwendung der diagnostischen Vorrichtung die zu untersuchende Körperflüssigkeit aufnehmen kann. Außerdem ist wichtig, daß die erwähnte starke Bindung zwischen dem Träger und der Farbstoffkomponente des Stoffgemisches eintritt.

Erfindungsgemäß wird die diagnostische Vorrichtung vorzugsweise unter stark vermindertem Druck licht-, feuchtigkeits- und luftdicht verpackt, was zweckmäßigerweise unmittelbar nach dem Gefriertrocknen noch in der Gefriertrocknungsvorrichtung geschieht. Ein Zutritt von Luft und Feuchtigkeit kann aber auch auf andere Art vermieden werden. Beispielsweise kann man nach dem Trocknen die Verpackungshülle mit der darin befindlichen diagnostischen Vorrichtung mit trockenem Stickstoff füllen und verschließen. Auch ist es möglich, in der Verpackungshülle außer der diagnostischen Vorrichtung ein Trockenmittel, z. B. ein Molekularsieb, anzuordnen.

**Patentansprüche**

1. Verfahren zum Herstellen einer diagnostischen Vorrichtung zum Nachweis einer pathologisch erhöhten Konzentration von Dehydrogenasen in Flüssigkeiten von Menschen, Tieren oder Pflanzen, die aus einem Träger und einem Stoffgemisch besteht, wobei das Stoffgemisch in flüssiger Form und in saurem pH-Wert mit dem Träger in einer UV-Strahlung ausschließenden Umgebung in Kontakt gebracht, getrocknet und dann in einer für Licht, das energiereicher als Rotlicht ist, undurchlässigen Verpackungshülle eingeschlossen wird, dadurch gekennzeichnet, daß die Kombination aus Träger und Stoffgemisch bis auf einen Feuchtigkeitsgehalt unter 1 % getrocknet und in der Verpackungshülle gegen Zutritt von Luft und Feuchtigkeit gesichert verschlossen wird.

2. Verfahren nach Anspruch 1, wobei ein Träger, der einen Redox-Farbstoff bindet, sowie ein Stoffgemisch aus dem der jeweiligen Dehydrogenase entsprechenden Substrat, einer wasserstoffübertragenden Verbindung, mindestens einem Redox-Farbstoff und gegebenenfalls einem Puffersystem eingesetzt werden, dadurch gekennzeichnet, daß der Träger polare Gruppen aufweist sowie das Stoffgemisch auf einen pH-Wert von unter 5,0 eingestellt wird.

3. Verfahren nach Anspruch 1 oder 2, dadurch gekennzeichnet, daß als Träger ein Cellulosefasern enthaltender Tampon eingesetzt wird.

4. Verfahren nach einem der vorstehenden Ansprüche, dadurch gekennzeichnet, daß die Kombination aus dem Träger und dem Stoffgemisch bereits vor dem Trocknen in der Verpackungshülle angeordnet wird.

5. Verfahren nach einem der vorstehenden Ansprüche, dadurch gekennzeichnet, daß die Verpackungshülle in der Trocknungsvorrichtung verschlossen wird.

6. Verfahren nach einem der Ansprüche 3 bis 5, dadurch gekennzeichnet, daß der Tampon vor dem Aufbringen des Stoffgemisches in einem Tamponapplikator angeordnet wird.

7. Verfahren nach Anspruch 6, dadurch gekennzeichnet, daß der Applikator eine Einführhülse mit einer flüssigkeitsundurchlässigen Innenwand aufweist und der Tampon derart in der Einführhülse angeordnet wird, daß innerhalb der Einführhülse vor dem Einführende des Tampons ein freier Raum gebildet wird.

8. Verfahren nach Anspruch 7, dadurch gekennzeichnet, daß eine Lösung des Stoffgemisches in den freien Raum eingespritzt wird.

9. Verfahren nach einem der vorstehenden Ansprüche, dadurch gekennzeichnet, daß als Verpackungshülle ein einseitig offenes, aus Metall hoher Wärmeleitfähigkeit bestehendes Röhrchen mit einem elastischen Verschlußstopfen eingesetzt wird.

10. Verfahren nach Anspruch 9, dadurch gekennzeichnet, daß das Röhrchen mit der Kombination aus Applikator und Tampon beschickt, dann das Stoffgemisch auf dem Tampon aufgebracht sowie anschließend das Röhrchen mit dem Verschlußstopfen teilweise verschlossen und mit seinem Inhalt getrocknet wird.

11. Verfahren nach einem der vorstehenden Ansprüche, dadurch gekennzeichnet, daß der Träger und das Stoffgemisch in einer Gefriertrocknungsvorrichtung getrocknet werden und dort anschließend die Verpackungshülle unter stark vermindertem Druck verschlossen wird.

12. Verfahren nach Anspruch 11, dadurch gekennzeichnet, daß in einer ersten Stufe bei einem geringeren Unterdruck und dann in einer zweiten Stufe bei einem größeren Unterdruck getrocknet wird.

**Claims**

1. Process for the production of a diagnostic device for the detection of a pathologically increased concentration of dehydrogenases in fluids of humans, animals or plants, which consists of a carrier and a substance mixture, the substance mixture being brought into contact, in liquid form and at an acid pH with the carrier in an environment which excludes UV radiation, dried and then enclosed in a packaging casing which is opaque to light of higher energy than red light, characterised in that the combination of carrier and substance mixture is dried to a moisture content of less than 1 % and is securely sealed in the packaging casing against the entry of air and moisture.

2. Process according to claim 1, in which a carrier, which bonds a redox dyestuff, and a substance mixture of the substrate corresponding to the particular dehydrogenase, a hydrogen donor compound, at least one redox dyestuff and, if appropriate, a buffer system are used, characterised in that the carrier has polar groups and the substance mixture is adjusted to a pH value of below 5.0.

3. Process according to claim 1 or 2, characterised in that a tampon containing cellulose fibers is used as the carrier.

4. Process according to one of the preceding claims, characterised in that the combination of the carrier and the substance mixture is already located in the packaging casing before drying.

5. Process according to one of the preceding claims, characterised in that the packaging casing is sealed in the drying device.

6. Process according to one of claims 3 to 5, characterised in that the tampon is located in a tampon applicator before application of the substance mixture.

7. Process according to claim 6, characterised in that the applicator has an insertion cartridge with an inner wall which is impermeable to liquid and the tampon is arranged in the insertion cartridge such that a free space is obtained within the insertion cartridge in front of the insertion end of the tampon.

8. Process according to claim 7, characterised in that a solution of the substance mixture is injected into the free space.

9. Process according to one of the preceding claims, characterized in that a small tube which is open on one side, consists of metal of high heat conductivity and has an elastic sealing plug is used as the packaging casing.

10. Process according to claim 9, characterised in that the small tube is charged with the combination of applicator and tampon, the substance mixture is then applied to the tampon and the small tube is subsequently partly sealed with the sealing plug and dried, together with its contents.

11. Process according to one of the preceding claims, characterised in that the carrier and the substance mixture are dried in a freeze-drying device and the packaging casing is then sealed in this device under greatly reduced pressure.

12. Process according to claim 11, characterised in that drying is carried out in a first stage under a relatively slightly reduced pressure and then in a second stage under a more greatly reduced pressure.

**Revendications**

1. Procédé de fabrication d'un dispositif diagnostique pour la détection d'une concentration pathologiquement accrue de déshydrogénases dans des liquides humains, d'animaux ou de plantes, qui est constitué par un support et un mélange de substances, le mélange de substances étant mis en contact, à l'état liquide et à une valeur de pH acide, avec le support dans un milieu à l'abri du rayonnement UV, séché et ensuite enfermé dans une gaine d'emballage opaque à de la lumière plus énergétique que la lumière rouge, caractérisé en ce que la combinaison du support et du mélange de substances est séchée jusqu'à une teneur en humidité inférieure à 1 % et scellée dans la gaine d'emballage, protégée contre l'entrée d'air et d'humidité.

2. Procédé selon la revendication 1, dans lequel on met en œuvre un support qui fixe un colorant redox, ainsi qu'un mélange de substances constitué du substrat correspondant à la déshydrogénase concernée, d'un composé fonctionnant comme vecteur d'hydrogène, d'au moins un colorant redox et, le cas échéant, d'un système tampon, caractérisé en ce que le support comporte des groupes polaires et que le mélange de substances est ajusté à une valeur de pH inférieure à 5,0.

3. Procédé selon la revendication 1 ou 2, caractérisé en ce qu'on met en œuvre comme support un tampon contenant des fibres de cellulose.

4. Procédé selon l'une des revendications précédentes, caractérisé en ce que la combinaison du support et du mélange de substances est déjà placée dans la gaine d'emballage avant le séchage.

5. Procédé selon l'une des revendications précédentes, caractérisé en ce que la gaine d'emballage est scellée dans l'installation de séchage.

6. Procédé selon l'une des revendications 3 à 5, caractérisé en ce que le tampon est placé dans un applicateur de tampon avant l'application du mélange de substances.

7. Procédé selon la revendication 6, caractérisé en ce que l'applicateur comporte une cartouche d'introduction avec une paroi interne imperméable aux liquides et que le tampon est disposé dans la cartouche d'introduction de telle manière qu'à l'intérieur de la cartouche il se forme un espace libre devant l'extrémité d'insertion du tampon.

8. Procédé selon la revendication 7, caractérisé en ce qu'une solution du mélange de substances est injectée dans l'espace libre.

9. Procédé selon l'une des revendications précédentes, caractérisé en ce qu'on met en œuvre

comme gaine d'emballage un petit tube métallique à conductibilité thermique élevée ouvert d'un côté, comportant un bouchon d'obturation élastique.

10. Procédé selon la revendication 9, caractérisé en ce que le petit tube est rempli de la combinaison de l'applicateur et du tampon, que le mélange de substances est ensuite appliqué sur le tampon et qu'aussitôt après le petit tube est partiellement obturé avec le bouchon d'obturation et séché avec son contenu.

11. Procédé selon les revendications précédentes, caractérisé en ce que le support et le mélange de substances sont séchés dans une installation de lyophilisation et que la gaine d'emballage y est ensuite scellée sous une pression fortement réduite.

12. Procédé selon la revendication 11, caractérisé en ce que, dans une première étape, on sèche sous un faible vide et qu'ensuite, dans une deuxième étape, on sèche sous un vide plus poussé.